# DEMANDE DE BREVET EUROPEEN

(11) **EP 4 043 929 A1**
(43) Date de publication de la demande: **17.08.2022**
(21) Numéro de dépôt: 22155681.4
(22) Date de dépôt: 08.02.2022
(51) Int. Cl.: G01T 1/20, H01L 27/146, H04N 5/369

(54) **DETECTEUR NUMERIQUE A ETAGES DE CONVERSION SUPERPOSES**

(30) Priorité: 11.02.2021 FR 2101326
(71) Demandeur: Trixell, 38430 Moirans (FR)
(72) Inventeur: BOSSET, Bruno, 38430 MOIRANS (FR); DUCOURANT, Thierry, 38430 MOIRANS (FR); COMMERE, Bruno, 38430 MOIRANS (FR)
(74) Mandataire: Marks & Clerk France

(57) **Abrégé**

L'invention concerne un détecteur numérique (100) comprenant :
- un bloc de conversion (110) destiné à convertir un rayonnement incident en charges électriques ;
- une carte électronique (50) assurant la conversion des charges électriques en une image digitale, le bloc de conversion (110) comprenant N étages de conversion (111-1, 111-2, 111-3, 111-4) superposés les uns sur les autres, N étant un nombre entier compris entre 2 et M, chacun des N étages de conversion comprenant :
∘ un substrat monolithique ;
∘ un premier ensemble convertisseur (133) sous forme d'une matrice polygonale, M étant le nombre de côtés de la matrice polygonale, M étant préférentiellement égal à 4, et configurée de façon à générer les charges électriques en fonction du rayonnement incident ;
∘ un module (114) d'adressage et de pilotage de la matrice, ledit module d'adressage et de pilotage étant disposé sur le substrat monolithique le long d'un côté de la matrice polygonale ;
chacun des N étages de conversion (111-1, 111-2, 111-3, 111-4) étant orienté d'au moins 1/M-ème de tour par rapport aux N-1 autres étages de conversion du bloc de conversion (110), et d'orientation distincte des N-1 autres étages de conversion du bloc de conversion (110).

## Description

L'invention se situe dans le domaine technique de l'imagerie médicale, et plus précisément dans celui des capteurs d'images numériques pour la radiologie. Elle concerne un détecteur numérique à étages de conversion superposés.

Un détecteur d'images traditionnel comprend typiquement un capteur plan comprenant un ensemble de pixels organisé en matrice selon des lignes et des colonnes, des blocs d'adressage de lignes, des blocs de lecture de colonnes, des conducteurs de ligne reliant les lignes de pixels à un bloc d'adressage de lignes, et des conducteurs de colonne reliant les colonnes de pixels à un bloc de lecture de colonnes. Il est à noter que, dans le cadre de la présente demande de brevet, les notions de colonne et de ligne n'ont qu'un sens relatif, une ligne de pixels et une colonne de pixels n'étant autres que des rangées de pixels disposées par exemple, et de manière non-limitative, perpendiculairement l'une à l'autre. Un conducteur de ligne, respectivement de colonne, est défini comme étant orienté parallèlement à une ligne de pixels, respectivement une colonne de pixels.

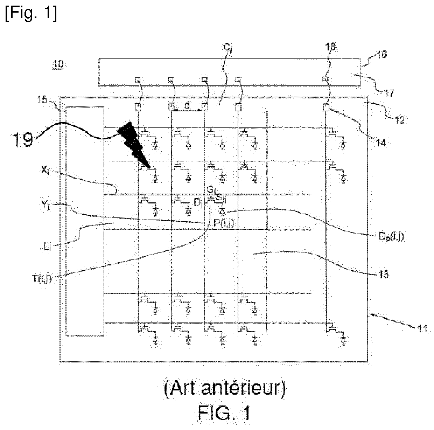

La figure 1 représente un détecteur d'images 10 traditionnel. Le détecteur d'images 10 comprend un capteur 11 réalisé sur un premier substrat monolithique 12. Le premier substrat monolithique 12 comprend un ensemble de pixels P(i,j) organisé en matrice 13 selon des lignes Li et des colonnes Cj. La matrice 13 peut comporter un nombre quelconque de lignes et de colonnes formant ainsi des pixels P(i,j). La matrice 13 forme une zone géographique sur le premier substrat 12. On note les pixels sous la forme générique P(i,j), où i et j sont des entiers naturels désignant respectivement le rang de la ligne et le rang de la colonne dans la matrice 13. L'ensemble des pixels P(i,j) est configuré de façon à générer des signaux en fonction d'un rayonnement frappant le détecteur 10. Plus précisément, un rayonnement incident 19 est converti par un scintillateur en un signal lumineux, et c'est ce signal lumineux qui est traité par l'ensemble des pixels. Le capteur 11 comprend des conducteurs de colonne Yj, chacun reliant les pixels d'une même colonne Cj. Les conducteurs de colonnes Yj sont destinés à transporter les signaux générés par les pixels P(i,j). De même, le capteur 11 comprend des conducteurs de ligne Xi, chacun reliant les pixels d'une même ligne Li. La matrice 13 de pixels P(i,j) comporte des colonnes Cj. De même, la matrice 13 de pixels P(i,j) comporte des lignes Li. Le capteur 10 comprend des plots de contact 14 situés à la périphérie du premier substrat 12 et en dehors de la matrice 13 de pixels P(i,j). Les plots de contact 14 sont reliés aux conducteurs de colonne Yj. Le détecteur d'images 10 comprend un bloc d'adressage de lignes 15 situé à proximité du premier substrat 12 et relié aux conducteurs de lignes Xi. On appelle bloc d'adressage de lignes 15 tout ensemble comportant au moins un bloc d'adressage de lignes. Le bloc 15 peut être intégré au premier substrat 12, comme représenté sur la figure 1, ou bien intégré à un substrat distinct. Le bloc d'adressage de lignes 15 permet d'adresser individuellement chaque ligne de pixels Li. Le détecteur d'images 10 comprend un bloc de lecture de colonnes 16 généralement réalisé sur un second substrat 17 différent du premier substrat 12. Le bloc de lecture de colonnes 16 comprend des points de connexion 18 reliant le bloc de lecture de colonnes 16 aux plots de contact 14. Le bloc de lecture de colonnes 16 permet de lire les signaux générés par les pixels de la ligne sélectionnée par le bloc d'adressage de lignes.

Un pixel P(i,j) comporte une photodiode Dp(i,j) associée à un interrupteur électronique T(i,j). Les photodiodes Dp(i,j) peuvent naturellement être remplacées par tout élément photosensible apte à générer un signal électrique lorsqu'il est soumis à un rayonnement de photons. La structure de pixel représentée sur la figure 1 est volontairement simplifiée et des structures plus complexes peuvent être mises en œuvre dans le cadre de l'invention.

L'interrupteur T(i,j) formé par un transistor est relié par sa grille Gi au conducteur de ligne Xi de la ligne i, par son drain Dj au conducteur de colonne Yj et par sa source Sij à la cathode de la photodiode Dp(i,j). Les anodes de toutes les photodiodes Dp(i,j) sont reliées à un potentiel commun, par exemple la masse. Le bloc d'adressage de ligne 15 comporte des éléments permettant de générer les signaux à injecter sur les conducteurs de ligne Xi pour piloter l'ouverture et la fermeture des transistors T(i,j). Le bloc de lecture de colonne 16 peut comporter des éléments permettant de traiter les signaux reçus sur les conducteurs de colonne Yj. En particulier, il peut s'agir d'un amplificateur et/ou d'un convertisseur analogique - numérique.

Traditionnellement, le capteur d'images 11 fonctionne de la manière suivante. Lors d'une phase de prise d'image, l'exposition des photodiodes Dp(i,j) à un rayonnement génère des charges électriques au niveau de la source Sij. La quantité de charges au niveau de chaque source Sij est fonction de l'intensité du rayonnement reçu par le pixel P(i,j) considéré. La phase de prise d'image est suivie d'une phase de lecture effectuée ligne par ligne. Les signaux injectés sur les différents conducteurs de ligne Xi passent successivement à l'état actif, de sorte que le potentiel de chaque conducteur de colonne Yj est successivement représentatif de la quantité de charges électriques accumulées dans les différents pixels P(i,j) de la colonne j.

A noter que dans cet exemple et à titre illustratif, la conversion du rayonnement incident en charges électriques se fait par un scintillateur et des photodiodes. On peut parler d'un étage de conversion. Un tel étage de conversion du rayonnement incident en charges électriques peut être obtenu avec un photodétecteur.

Afin d'obtenir une sensibilité plus importante au rayonnement X auquel le détecteur est soumis, il est connu d'empiler plusieurs matrices et plusieurs scintillateurs. En effet, l'empilement de plusieurs matrices et de plusieurs scintillateurs permet de réaliser des capteurs offrant une résolution en énergie de l'image de rayons X en séparant les rayonnements les moins énergétiques qui sont absorbés dans les couches supérieures du scintillateur et les plus énergétiques qui sont absorbés dans les couches plus profondes.

L'association et la conception judicieuse d'un ou plusieurs scintillateurs et d'une ou plusieurs matrices de photodiodes permet ainsi de réaliser des images dans lesquelles les rayonnements de différentes énergies sont séparés, à la manière des images "en couleurs" dans le domaine des caméras et téléviseurs fonctionnant dans le domaine visible. Dans la disposition la plus courante, les capteurs offrant cette résolution spectrale sont constitués de deux matrices de photodiodes associées à une ou plusieurs couches scintillatrices.

Si les dispositifs existants et déjà décrits offrent une large variété de structures et d'empilements, les moyens d'adressage et de lecture des matrices sont peu ou pas détaillés. Pourtant cette connexion avec l'extérieur est indispensable pour pouvoir lire le signal généré au niveau des pixels et transmettre un signal électronique autorisant la construction d'une image numérique. Dans les dispositions les plus courantes de l'art antérieur, l'adressage et la lecture sont réalisés sur deux bords perpendiculaires de la matrice, l'adressage étant usuellement réalisé par les lignes et la lecture par les colonnes.

Si deux bords sont nécessaires pour le pilotage et la lecture de chaque matrice de pixels, tel que cela a été présenté dans l'exemple de détecteur de l'art antérieur de la figure 1, il devient clair qu'un dispositif d'imagerie spectrale carré ou rectangulaire pourra être assez facilement réalisé en empilant deux matrices et en utilisant ainsi les quatre côtés de l'empilement pour les fonctions de pilotage et de lecture des deux matrices. Toutefois, si l'empilement comporte plus de deux matrices de photodiodes, cette connexion devient beaucoup délicate et nécessite des architectures complexes. Si, de plus, le capteur est contraint en épaisseur comme par exemple dans le cas des détecteurs portables qui doivent s'inscrire dans un gabarit de dimensions normalisées, la réalisation devient très difficile.

L'invention vise à pallier tout ou partie des problèmes cités plus haut en proposant un détecteur d'images radiologique permettant de maximiser l'absorption des rayons X, la résolution spatiale et spectrale de l'image et la sélectivité entre les différentes énergies, tout en restant peu encombrant. Un tel détecteur permet d'obtenir des empilements pouvant comporter une pluralité de matrices de photodiodes et potentiellement deux fois plus de couches scintillatrices tout en conservant la possibilité de piloter et de lire simplement les matrices.

A cet effet, l'invention a pour objet un détecteur numérique comprenant :
- un bloc de conversion destiné à convertir un rayonnement incident en charges électriques ;
- une carte électronique assurant la conversion des charges électriques en une image digitale, caractérisé en ce que le bloc de conversion comprend N étages de conversion superposés les uns sur les autres, N étant un nombre entier compris entre 2 et M, chacun des N étages de conversion comprenant :
   o un substrat monolithique ;
   o un premier ensemble convertisseur sous forme d'une matrice polygonale, M étant le nombre de côtés de la matrice polygonale, M étant préférentiellement égal à 4, ladite matrice étant disposée sur le substrat monolithique, et étant organisée selon des lignes et des colonnes et configurée de façon à générer les charges électriques en fonction du rayonnement incident ;
   o un module d'adressage et de pilotage de la matrice, ledit module d'adressage et de pilotage étant disposé sur le substrat monolithique en pied des colonnes le long d'un côté de la matrice polygonale, et destiné à adresser les lignes et transporter les charges électriques générées dans les colonnes ;
   et en ce que chacun des N étages de conversion est orienté d'au moins 1/M-ème de tour par rapport aux N-1 autres étages de conversion du bloc de conversion, et d'orientation distincte des N-1 autres étages de conversion du bloc de conversion.

Dans un mode de réalisation de l'invention, lesdits ensembles convertisseurs sont des photodétecteurs, chacun des photodétecteurs étant apte à absorber le rayonnement incident et à le transformer en charges électriques.

Avantageusement, au moins un des N étages de conversion comprend en outre un deuxième ensemble convertisseur disposé sous le substrat monolithique dudit au moins un des N étages de conversion.

Avantageusement, le détecteur numérique selon l'invention comprend en outre un filtre du rayonnement incident d'énergie prédéterminée entre le deuxième ensemble convertisseur du au moins un des N étages de conversion et le premier ensemble convertisseur de l'étage inférieur de conversion.

Dans un autre mode de réalisation de l'invention, chacun desdits ensembles convertisseurs comprend un premier scintillateur apte à convertir le rayonnement incident en un signal lumineux, et dans lequel la matrice polygonale est une matrice de pixels et configurée de façon à générer les charges électriques en fonction du signal lumineux émis par le premier scintillateur disposé sur la matrice polygonale.

Avantageusement, au moins un des N étages de conversion comprend en outre un deuxième scintillateur disposé sous le substrat monolithique dudit au moins un des N étages de conversion.

Avantageusement, le détecteur numérique selon l'invention comprend en outre un filtre du rayonnement incident de faible énergie entre le deuxième scintillateur du au moins un des N étages de conversion et le premier scintillateur de l'étage inférieur de conversion.

Avantageusement, au moins un pixel de la matrice polygonale de pixels d'au moins un des N étages de conversion comprend des photodiodes comportant deux électrodes transparentes.

Avantageusement, les pixels de la matrice polygonale de pixels de chacun des N étages de conversion comprennent des photodiodes comportant une électrode transparente et une électrode opaque, disposées en alternance selon une disposition en damier dans la matrice polygonale d'un étage de conversion et en damier d'un étage de conversion à l'étage de conversion adjacent.

Avantageusement, pour au moins un des N étages de conversion, le module d'adressage et de pilotage de la matrice est reporté directement sur la matrice.

Avantageusement, pour ledit au moins un des N étages de conversion, la matrice polygonale comprend des extensions portant des conducteurs reliant le module d'adressage et de pilotage à la matrice polygonale.

L'invention sera mieux comprise et d'autres avantages apparaîtront à la lecture de la description détaillée d'un mode de réalisation donné à titre d'exemple, description illustrée par le dessin joint dans lequel :
[Fig.1] La figure 1 représente schématiquement un détecteur d'images traditionnel;
[Fig.2] La figure 2 représente schématiquement le principe d'un détecteur numérique à étages de conversion superposés selon l'invention ;
[Fig.3] La figure 3 représente schématiquement un premier mode de réalisation d'un détecteur numérique selon l'invention ;
[Fig.4] La figure 4 représente schématiquement un
deuxième mode de réalisation d'un détecteur numérique selon l'invention ;
[Fig.5] La figure 5 représente schématiquement un troisième mode de réalisation d'un détecteur numérique selon l'invention ;
[Fig.6] La figure 6 représente schématiquement un quatrième mode de réalisation d'un détecteur numérique selon l'invention ;
[Fig.7] La figure 7 représente schématiquement un cinquième mode de réalisation d'un détecteur numérique selon l'invention ;
[Fig.8] La figure 8 représente schématiquement un sixième mode de réalisation d'un détecteur numérique selon l'invention ;
[Fig.9] La figure 9 représente schématiquement un septième mode de réalisation d'un détecteur numérique selon l'invention.

Sur ces figures, dans un souci de clarté, les échelles ne sont pas respectées. Par ailleurs, les mêmes éléments porteront les mêmes repères dans les différentes figures.

La figure 1 représente schématiquement un détecteur d'images traditionnel. Elle a été présentée en introduction.

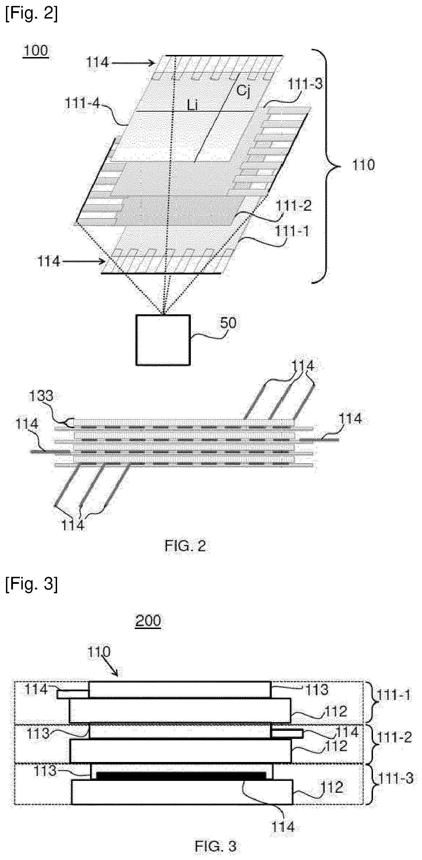

La figure 2 représente schématiquement le principe d'un détecteur numérique 100 à étages de conversion superposés selon l'invention. Le détecteur numérique 100 comprend un bloc de conversion 110 destiné à convertir un rayonnement incident en charges électriques, et une carte électronique 50 assurant la conversion des charges électriques en une image digitale. Selon l'invention, le bloc de conversion 110 comprend N étages de conversion 111-1, 111-2, 111-3, 111-4 superposés les uns sur les autres. N est un nombre entier compris entre 2 et M, et préférentiellement au moins trois. Chacun des N étages de conversion comprend un substrat monolithique 112, un premier ensemble convertisseur 113 sous forme d'une matrice polygonale, M étant le nombre de côtés de la matrice polygonale, ladite matrice étant disposée sur le substrat monolithique, et étant organisée selon des lignes Li et des colonnes Cj et configurée de façon à générer les charges électriques en fonction du rayonnement incident. Chacun des N étages de conversion comprend en outre un module 114 d'adressage et de pilotage de la matrice, ledit module d'adressage et de pilotage étant disposé sur le substrat monolithique 112 en pied des colonnes Cj le long d'un côté de la matrice polygonale, et destiné à adresser les lignes Li et transporter les charges électriques générées dans les colonnes Cj. Chacun des N étages de conversion 111-1, 111-2, 111-3, 111-4 est orienté d'au moins 1/M-ème de tour par rapport aux N-1 autres étages de conversion du bloc de conversion 110, et d'orientation distincte des N-1 autres étages de conversion du bloc de conversion 110. Autrement dit, les étages de conversion sont superposés les uns aux autres selon un premier axe, et chacun des étages est orienté, autour du premier axe, selon un angle égal à un multiple de 1/M, et de sorte que les modules 114 d'adressage et de pilotage de la matrice des N étages de conversion soient répartis, chacun sur un côté du bloc de conversion.

La matrice polygonale a M côtés. M est préférentiellement égal à 4, auquel cas la matrice est rectangulaire (ou carrée). L'invention s'applique également pour des valeurs différentes de M, sous réserve que le module 114 d'adressage et de pilotage de la matrice associé soit disposé sur un bord de la matrice, par exemple M égal à 3, 5, 6 voire plus. Dans ce qui suit, nous détaillerons l'invention avec une matrice rectangulaire, c'est-à-dire avec M égal à 4.

Comme représenté sur la figure 2, le bloc de conversion 110 comprend quatre étages de conversion 111-1, 111-2, 111-3, 111-4. Chacun des étages de conversion est orienté d'1/4 par rapport à un autre étage de conversion, et a une orientation distincte par rapport aux trois autres étages de conversion. En d'autres termes, à chaque bord du bloc de conversion est associé un seul module 114 d'adressage et de pilotage de la matrice, bien que le bloc de conversion comprenne quatre étages de conversion.

En bas de la figure 2, l'empilement des étages de conversion est représenté avec les modules 114 d'adressage et de pilotage de la matrice. Dans cette illustration, la conversion du rayonnement incident en charges électriques est réalisée par un scintillateur et des photodiodes. Alternativement, la conversion du rayonnement incident en charges électriques peut être obtenue avec un photodétecteur.

Avantageusement, les matrices des étages de conversion sont réalisées sur substrat 112 mince. Un substrat mince est réalisé en plastique souple de quelques dizaines de micromètres d'épaisseur. Comme mentionné ci-dessus, pour que le pilotage et l'adressage puissent se faire par un seul côté du bloc de conversion, les modules 114 sont basés sur la technologie dite des "drivers intégrés" pour le pilotage (aussi connue sous l'acronyme GOA : Gate drivers On Array). Il est ainsi possible de réaliser des empilements pouvant comporter jusqu'à quatre matrices de photodiodes et jusqu'à huit couches de scintillateurs distinctes. Il devient alors possible de séparer l'énergie des rayons X incidents en plusieurs canaux (potentiellement jusqu'à 8 dans l'exemple où M est égal à 4) tout en améliorant la sensibilité et la résolution de l'ensemble. De tels modules 114 sont par exemple décrits dans les documents EP2708023B1 et EP3672230A1. Ces modules connecteurs (par exemple de type FPC : Flexible Printed Circuit, incluant un circuit intégré de lecture) peuvent être assemblés par la technique classique de flexage avec un film conducteur anisotrope (ACF). Une variante peut être obtenue en reportant les circuits intégrés de lecture directement sur le même substrat par la technologie flip-chip et réalisant des extensions du substrat sur un bord au-delà de ces circuits. Il est alors possible de connecter directement les matrices à une carte électronique avec un connecteur simple de type zif ou équivalent. Cet aspect sera détaillé plus loin.

Ainsi, l'invention permet de réaliser un empilement des étages de conversion permettant de réaliser un capteur d'image radiologique avec la capacité de produire simultanément des images correspondant à différentes énergies des photons X incidents. Il est possible d'obtenir jusqu'à huit canaux énergétiques sans augmenter notablement la complexité de l'assemblage par rapport à des produits qui actuellement ne pourraient séparer que haute et basse énergie. L'ensemble de l'empilement peut être réalisé en étant compatible avec les contraintes dimensionnelles imposées pour les capteurs plans portables.

Cet aspect est novateur car il permet de surmonter à la fois les problèmes de connectiques associés à un empilement d'étages de conversion et de limiter l'absorption des rayons X dans les substrats. Au contraire il augmente l'absorption globale de l'assemblage puisqu'on superpose plusieurs scintillateurs. En effet, dans l'art antérieur, la question de la connexion des matrices est rarement abordée et il n'existe pas de solution simple pour connecter les circuits de pilotage et les circuits de lecture en périphérie lorsque l'empilement comporte plus de deux matrices. Même s'ils restent possibles d'un point de vue théorique, les empilements comportant plus de trois scintillateurs sont difficilement réalisables sans recourir à une architecture complexe et encombrante. Dans l'art antérieur, lorsque le nombre de matrices dépasse deux, il devient nécessaire d'avoir plusieurs rangs de connecteurs sur au moins un côté, ce qui pose des problèmes difficiles pour fabriquer et assembler le produit final. Il faudrait dans ce cas avoir des dispositifs entrelacés ou emboités peu compatibles avec une production industrielle. Cette difficulté est accrue lorsqu'il s'agit de détecteurs portables où les contraintes de masse, d'épaisseur et d'encombrement latéral sont particulièrement sévères. Cette difficulté dissuade l'Homme du métier à envisager un tel empilement synonyme de complexité de réalisation et d'encombrement supérieur. De plus l'empilement d'un nombre élevé de matrices habituellement réalisées sur des substrats épais augmente à la fois l'absorption des rayons X perdue dans les substrats et leur diffusion latérale, ce qui entraine une limitation intrinsèque de la DQE (Detective Quantum Efficiency). Dans la pratique, les architectures actuelles sont donc limitées à l'obtention d'images en double énergie (basse et haute énergie), chaque canal étant associé à une matrice de l'empilement. L'invention apporte une solution au préjugé selon lequel un empilement d'étages de conversion signifie encombrement et complexité de la connectique et diffusion latérale de lumière entre les couches et dégradation de la résolution. L'architecture du détecteur selon l'invention répond à ces problématiques. La structure composite ainsi réalisée peut être assemblée simplement dans un capteur sans apporter de complexité particulière par rapport au cas d'une simple matrice. L'augmentation du nombre de couches permet de séparer l'épaisseur totale du scintillateur en tranches de fines épaisseurs. L'épaisseur totale de ces couches permet d'obtenir une absorption élevée, également notée DQE(0) tout en améliorant la MTF en particulier sur la ou les premières couches. Il est également envisageable d'avoir des pixels de tailles et de formes différentes selon les couches pour optimiser la performance globale (sensibilité, résolution spatiale, résolution spectrale) ou pour favoriser l'une de ces caractéristiques selon le type d'application visée.

La figure 3 représente schématiquement un premier mode de réalisation d'un détecteur numérique 200 selon l'invention. Dans ce mode de réalisation, les ensembles convertisseurs 113 sont des photodétecteurs, chacun des photodétecteurs étant apte à absorber le rayonnement incident et à le transformer en charges électriques.

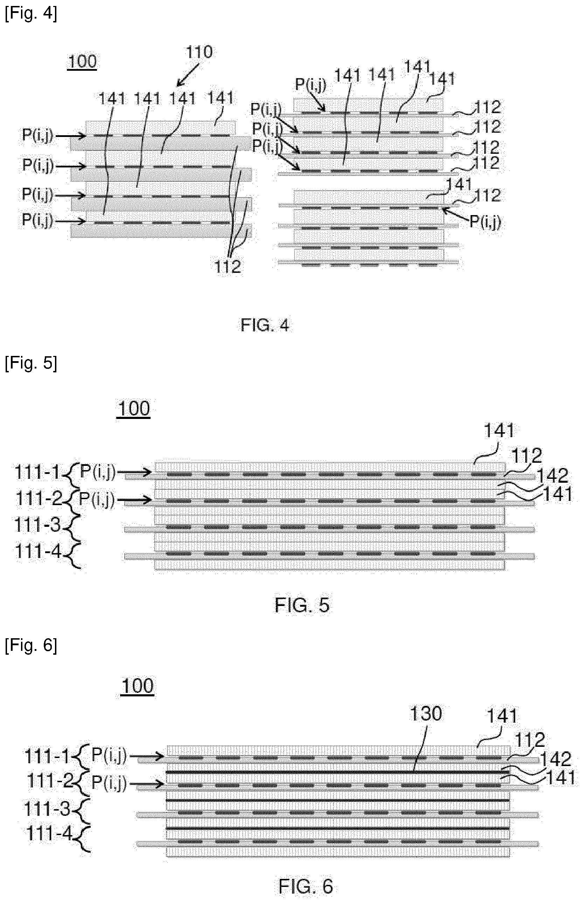

La figure 4 représente schématiquement un deuxième mode de réalisation d'un détecteur numérique 100 selon l'invention. Dans ce mode de réalisation, chacun desdits ensembles convertisseurs 133 comprend un premier scintillateur 141 apte à convertir le rayonnement incident en un signal lumineux, et dans lequel la matrice polygonale est une matrice de pixels P(i,j) et configurée de façon à générer les charges électriques en fonction du signal lumineux 19 émis par le premier scintillateur 141 disposé sur la matrice polygonale.

La figure 5 représente schématiquement un troisième mode de réalisation d'un détecteur numérique 100 selon l'invention. Dans ce mode de réalisation, au moins un des N étages de conversion 111-1, 111-2, 111-3, 111-4 comprend en outre un deuxième scintillateur 142 disposé sous le substrat monolithique 112 dudit au moins un des N étages de conversion 111-1, 111-2, 111-3, 111-4.

Dans l'exemple de la figure 5, tous les étages de conversion comprennent un deuxième scintillateur 142 sous le substrat 112. Chaque matrice de photodiodes est associée à deux scintillateurs. Cette structure peut être avantageuse si tout ou partie des photodiodes ont deux électrodes transparentes, permettant ainsi de maximiser l'efficacité de collecte de la lumière.

Cette caractéristique de l'invention s'applique aussi dans le cas où les ensembles convertisseurs sont des photodétecteurs. Dans une telle réalisation, au moins un des N étages de conversion 111-1, 111-2, 111-3, 111-4 comprend alors un deuxième ensemble convertisseur disposé sous le substrat monolithique 112 dudit au moins un des N étages de conversion 111-1, 111-2, 111-3, 111-4.

La figure 6 représente schématiquement un quatrième mode de réalisation d'un détecteur numérique 100 selon l'invention. Dans ce mode de réalisation, le détecteur numérique 100 comprend en outre un filtre 130 du rayonnement incident d'énergie prédéterminée entre le deuxième scintillateur 142 du au moins un des N étages de conversion 111-1, 111-2, 111-3, 111-4 et le premier scintillateur 141 de l'étage inférieur de conversion. Le niveau d'énergie est situé entre 20 et 150 keV. Dans le domaine de la radiologie, il est généralement entre 40 et 120 keV. Les photons les plus énergétiques (dans la partie haute des plages précédemment citées) accèdent aux couches profondes du bloc de conversion, alors que les photons d'énergie faible (dans la partie basse des plages précédemment citées) sont absorbés dans les premières couches du bloc de conversion. Le filtre 130 est destiné à filtrer les rayons X de plus faible énergie, permettant ainsi de durcir le spectre et d'augmenter la sélectivité entre les couches successives. Ce durcissement a pour conséquence de réduire l'absorption totale par l'ensemble des couches scintillatrices mais peut présenter un avantage lorsque la sélectivité spectrale est préférée à la sensibilité totale.

Cette caractéristique de l'invention s'applique aussi dans le cas où les ensembles convertisseurs sont des photodétecteurs. Dans une telle réalisation, le détecteur numérique 200 comprend en outre un filtre 130 du rayonnement incident d'énergie prédéterminée entre le deuxième ensemble convertisseur du au moins un des N étages de conversion 111-1, 111-2, 111-3, 111-4 et le premier ensemble convertisseur 113 de l'étage inférieur de conversion 111-1, 111-2, 111-3, 111-4.

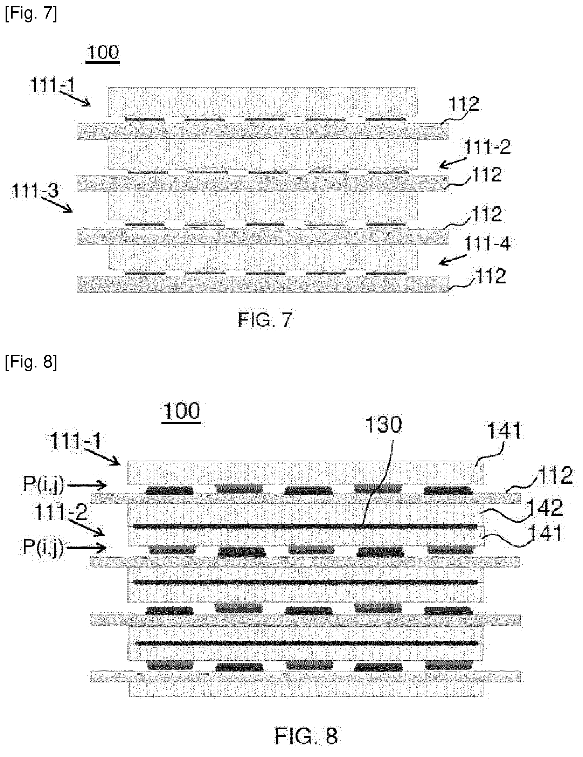

La figure 7 représente schématiquement un cinquième mode de réalisation d'un détecteur numérique 100 selon l'invention. Dans ce cinquième mode de réalisation, au moins un pixel P(i,j) de la matrice polygonale de pixels P(i,j) d'au moins un des N étages de conversion 111-1, 111-2, 111-3, 111-4 comprend des photodiodes comportant deux électrodes transparentes. Ce mode de réalisation présente l'avantage de maximiser l'efficacité de collecte de la lumière émise par les scintillateurs puisque les photodiodes peuvent recevoir des photons visibles par le haut et par le bas. La contrepartie peut être une diminution de la résolution spatiale (MTF) en particulier si les couches de substrats transparentes sont épaisses et autorisent une diffusion latérale de lumière. Il est donc préférable d'utiliser des substrats aussi minces que possible pour limiter cette diffusion et pour réduire également l'absorption des rayons X, bien que cette absorption soit généralement faible, de l'ordre de quelques pourcents.

La figure 8 représente schématiquement un sixième mode de réalisation d'un détecteur numérique 100 selon l'invention. Dans ce sixième mode de réalisation, les pixels P(i,j) de la matrice polygonale de pixels P(i,j) de chacun des N étages de conversion 111-1, 111-2, 111-3, 111-4 comprend des photodiodes comportant une électrode transparente et une électrode opaque, disposées en alternance selon une disposition en damier dans la matrice polygonale d'un étage de conversion et en damier d'un étage de conversion à l'étage de conversion adjacent (sur la figure 8, et comme cela est compris par un Homme du métier à la lecture de cette description, les traits clairs et sombres représentent les pixels et leur électrode opaque). Autrement dit, chaque matrice comporte des pixels dont une électrode est transparente et l'autre opaque, cette caractéristique étant alternée selon une disposition en damier. Cette configuration rend possible, sur la même matrice, par exemple pour l'étage 111-1, de collecter la lumière émise par le scintillateur inférieur 142 avec les pixels dont l'électrode inférieure est transparente et la lumière émise par le scintillateur supérieur 141 avec les pixels dont l'électrode supérieure est transparente. En associant ces matrices en damier avec huit scintillateurs, il est alors possible d'obtenir une image spectrale sur huit canaux d'énergie. De manière optionnelle, le spectre des rayons X peut avantageusement être durci avec des filtres 130 métalliques intermédiaires pour améliorer la sélectivité entre les canaux, au prix d'une moindre sensibilité.

L'alternance de transparence (face avant/face arrière) peut éventuellement se faire avec des pixels non carrés, pour recombiner une image double énergie à pixels carrés. Par exemple, un pixel rectangulaire basse énergie et un pixel rectangulaire haute énergie se recombinent en un seul pixel carré double énergie équivalent à deux couleurs. Cette alternance "en damier" des pixels transparents en face avant et face arrière peut se généraliser à d'autres combinaisons géométriques permettant d'alterner ces transparences sur les deux faces, avec par exemple une alternance de lignes ou colonnes paires/impaires ou d'autres répartitions éventuellement dissymétriques.

Dans un autre mode de réalisation de l'invention, pour au moins un des N étages de conversion 111-1, 111-2, 111-3, 111-4, le module 114 d'adressage et de pilotage de la matrice peut être reporté directement sur la matrice. Ce mode de réalisation est combinable avec les autres modes de réalisation présentés précédemment.

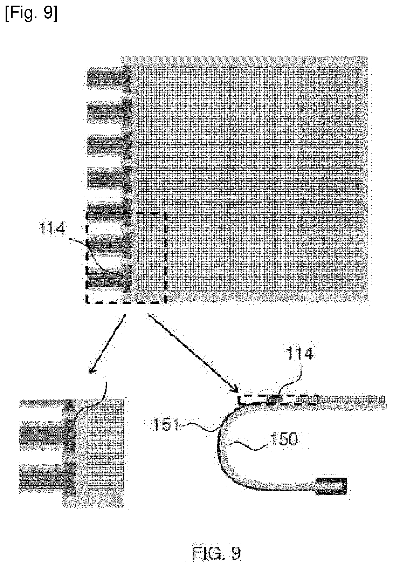

La figure 9 représente schématiquement un septième mode de réalisation d'un détecteur numérique selon l'invention. Dans ce mode de réalisation, avec le module 114 d'adressage et de pilotage de la matrice reporté directement sur la matrice, pour au moins un des N étages de conversion 111-1, 111-2, 111-3, 111-4, et avantageusement pour tous les étages de conversion, la matrice polygonale comprend des extensions 150 portant des conducteurs 151 reliant le module 114 d'adressage et de pilotage à la matrice polygonale.

Dans le cas de l'ensemble convertisseur comprenant un scintillateur et une matrice de photodiodes, la matrice polygonale de pixels P(i,j) comprend des extensions 150 portant des conducteurs 151 reliant le module 114 d'adressage et de pilotage des pixels P(i,j) aux pixels P(i,j) de à la matrice polygonale de pixels P(i,j).

Grâce au report direct des circuits de lecture du signal émis par les photodiodes, par exemple en technologie "chip on flex", il n'est plus nécessaire de flexer les connecteurs souples portant ces circuits (FPC). Cette caractéristique rend possible la connexion directe des matrices de photodiodes par la réalisation dans le même substrat des extensions 150 qui portent les conducteurs 151. L'ensemble de matrices et scintillateurs prolongé par ces languettes souples peut alors être relié directement à une carte électronique au moyen de connecteurs de type zif ou équivalent sans qu'il soit nécessaire de réaliser une opération de flexage sur la carte électronique.

On peut noter que cette dernière caractéristique de l'invention s'applique dans le cadre de l'invention. Toutefois, elle peut aussi s'appliquer au cas où le bloc de conversion comprend seulement un étage de conversion, c'est-à-dire sans empilement de matrice.

L'invention permet de réaliser des capteurs d'images radiologiques avec une sélectivité spectrale allant jusqu'à huit canaux alors que les systèmes actuels sont limités à deux niveaux d'énergie. L'empilement proposé, qui combine un substrat mince, l'adressage par drivers intégrés et éventuellement des filtres et des combinaisons avantageuses de photodiodes avec des électrodes opaques ou transparentes, permet de réaliser ces capteurs avec de nombreuses variantes dans un encombrement réduit compatible avec les exigences de réalisation d'un détecteur portable. Selon les variantes choisies, il est possible de maximiser l'absorption des rayons X, la résolution spatiale et spectrale de l'image et la sélectivité entre les différentes énergies.

Les applications de l'imagerie spectrale en radiologie sont actuellement restreintes à la double énergie et à la tomographie (scanner). Grâce à l'invention, la possibilité d'étendre la sélectivité spectrale peut ouvrir des champs importants pour la qualité du diagnostic en séparant différents tissus.

L'invention est décrite dans le domaine de la radiologie. Cependant, les applications industrielles en sécurité et contrôle non destructif sont également importantes et l'utilisation d'un capteur plan multi-spectral sur la base de l'invention pourrait également avantageusement remplacer des systèmes à capteur linéaires, par exemple pour le contrôle de bagages dans les aéroports.

## Revendications

1. Détecteur numérique (100, 200) comprenant :
- un bloc de conversion (110) destiné à convertir un rayonnement incident en charges électriques ;
- une carte électronique (50) assurant la conversion des charges électriques en une image digitale,
le bloc de conversion (110) comprenant N étages de conversion (111-1, 111-2, 111-3, 111-4) superposés les uns sur les autres, N étant un nombre entier compris entre 2 et M, chacun des N étages de conversion comprenant :
o un substrat monolithique (112) ;
o un premier ensemble convertisseur (113, 133) sous forme d'une matrice polygonale, M étant le nombre de côtés de la matrice polygonale, M étant préférentiellement égal à 4, ladite matrice étant disposée sur le substrat monolithique, et étant organisée selon des lignes (Li) et des colonnes (Cj) et configurée de façon à générer les charges électriques en fonction du rayonnement incident ;
o un module (114) d'adressage et de pilotage de la matrice, ledit module d'adressage et de pilotage étant disposé sur le substrat monolithique (112) en pied des colonnes (Cj) le long d'un côté de la matrice polygonale, et destiné à adresser les lignes (Li) et transporter les charges électriques générées dans les colonnes (Cj) ;
chacun des N étages de conversion (111-1, 111-2, 111-3, 111-4) étant orienté d'au moins 1/M-ème de tour par rapport aux N-1 autres étages de conversion du bloc de conversion (110), et d'orientation distincte des N-1 autres étages de conversion du bloc de conversion (110),
le détecteur numérique (100, 200) étant **caractérisé en ce que** chacun desdits ensembles convertisseurs (133) comprend un premier scintillateur (141) apte à convertir le rayonnement incident en un signal lumineux, et dans lequel la matrice polygonale est une matrice de pixels (P(i,j)) et configurée de façon à générer les charges électriques en fonction du signal lumineux (19) émis par le premier scintillateur (141) disposé sur la matrice polygonale,
et **en ce que** au moins un des N étages de conversion (111-1, 111-2, 111-3, 111-4) comprend en outre un deuxième scintillateur (142) disposé sous le substrat monolithique (112) dudit au moins un des N étages de conversion (111-1, 111-2, 111-3, 111-4).

2. Détecteur numérique (200) selon la revendication 1, dans lequel lesdits ensembles convertisseurs (113) sont des photodétecteurs, chacun des photodétecteurs étant apte à absorber le rayonnement incident et à le transformer en charges électriques.

3. Détecteur numérique (200) selon la revendication 2, dans lequel au moins un des N étages de conversion (111-1, 111-2, 111-3, 111-4) comprend en outre un deuxième ensemble convertisseur disposé sous le substrat monolithique (112) dudit au moins un des N étages de conversion (111-1, 111-2, 111-3, 111-4).

4. Détecteur numérique (200) selon la revendication 3, comprenant en outre un filtre (130) du rayonnement incident d'énergie prédéterminée entre le deuxième ensemble convertisseur du au moins un des N étages de conversion (111-1, 111-2, 111-3, 111-4) et le premier ensemble convertisseur (113) de l'étage inférieur de conversion (111-1, 111-2, 111-3, 111-4).

5. Détecteur numérique (100) selon la revendication 1, comprenant en outre un filtre (130) du rayonnement incident de faible énergie entre le deuxième scintillateur (142) du au moins un des N étages de conversion (111-1, 111-2, 111-3, 111-4) et le premier scintillateur (141) de l'étage inférieur de conversion.

6. Détecteur numérique (100) selon l'une quelconque des revendications 1 à 5, dans lequel au moins un pixel (P(i,j)) de la matrice polygonale de pixels (P(i,j)) d'au moins un des N étages de conversion (111-1, 111-2, 111-3, 111-4) comprend des photodiodes comportant deux électrodes transparentes.

7. Détecteur numérique (100) selon l'une quelconque des revendications 1 à 6, dans lequel les pixels (P(i,j)) de la matrice polygonale de pixels (P(i,j)) de chacun des N étages de conversion (111-1, 111-2, 111-3, 111-4) comprennent des photodiodes comportant une électrode transparente et une électrode opaque, disposées en alternance selon une disposition en damier dans la matrice polygonale d'un étage de conversion et en damier d'un étage de conversion à l'étage de conversion adjacent.

8. Détecteur numérique (100, 200) selon l'une quelconque des revendications 1 à 7, dans lequel, pour au moins un des N étages de conversion (111-1, 111-2, 111-3, 111-4), le module (114) d'adressage et de pilotage de la matrice est reporté directement sur la matrice.

9. Détecteur numérique (100, 200) selon la revendication 8, dans lequel, pour ledit au moins un des N étages de conversion (111-1, 111-2, 111-3, 111-4), la matrice polygonale comprend des extensions (150) portant des conducteurs (151) reliant le module (114) d'adressage et de pilotage à la matrice polygonale.
